(19) Europäisches Patentamt
European Patent Office
Office européen des brevets

(11) Veröffentlichungsnummer : **0 225 272 B1**

(12) # EUROPÄISCHE PATENTSCHRIFT

(45) Veröffentlichungstag der Patentschrift : **05.04.89**

(51) Int. Cl.⁴ : **C 07 J 1/00**, A 61 K 31/565// C07J17/00

(21) Anmeldenummer : **86730177.2**

(22) Anmeldetag : **29.10.86**

(54) **1-Methyl-15alpha-alkyl-androsta-1,4-dien-3,17-dione, Verf. zu deren Herstellung und diese enthaltende pharmazeutische Präparate.**

(30) Priorität : **01.11.85 DE 3539244**

(43) Veröffentlichungstag der Anmeldung : **10.06.87 Patentblatt 87/24**

(45) Bekanntmachung des Hinweises auf die Patenterteilung : **05.04.89 Patentblatt 89/14**

(84) Benannte Vertragsstaaten : **AT BE CH DE ES FR GB GR IT LI LU NL SE**

(56) Entgegenhaltungen :
**EP--A-- 0 129 500**
**US--A-- 3 766 224**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Kerb, Ulrich**
**Prinzregentenstrasse 7**
**D-1000 Berlin 31 (DE)**
Erfinder : **Nishino, Yukishige**
**Lanzendorfer Weg 14**
**D-1000 Berlin 22 (DE)**
Erfinder : **Henderson, David**
**Jahnstrasse 17**
**D-1000 Berlin 28 (DE)**

EP 0 225 272 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 1-Methyl-15α-alkyl-androsta-1,4-dien-3,17-dione, Verfahren zu deren Herstellung und diese enthaltende pharmazeutische Präparate.

Die neuen Verbindungen sind durch die allgemeine Formel I gekennzeichnet

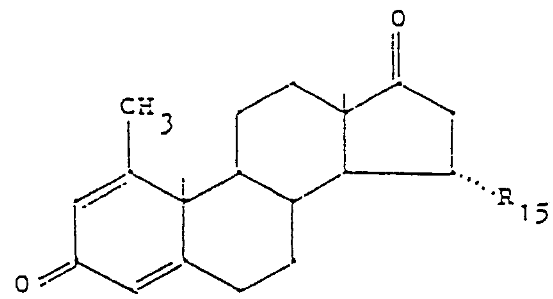

(I)

worin $R_{15}$ eine Alkylgruppe mit 1 bis 4 Kohlenstoffatomen darstellt, die gegebenenfalls in 1- und/oder 2-Stellung durch eine Hydroxy- oder $C_2$-$C_7$-Alkanoyloxygruppe oder eine 1,2-Isopropylidendioxygruppe substituiert ist.

Bevorzugte $R_{15}$-Gruppen sind :

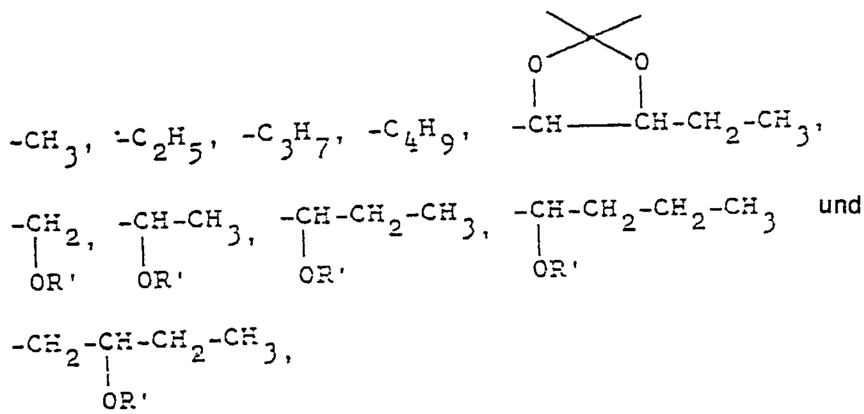

und

wobei R' für Wasserstoff oder eine Alkanoylgruppe mit 2 bis 7 Kohlenstoffatomen steht.

Als Alkanoylgruppen mit 2 bis 7 Kohlenstoffatomen sind die Acetyl-, Propionyl-, Butyryl- und Valerylgruppe besonders geeignet.

Es wurde nun gefunden, daß die neuen Verbindungen der allgemeinen Formel I als Aromatasehemmer die Östrogenbiosynthese hemmen.

Im Vergleich zu dem bekannten Aromatasehemmer 4-Hydroxy-4-androsten-3,17-dion wird die Serum-Östradiolkonzentration mit den erfindungsgemäßen Verbindungen stärker gesenkt.

Infantile weibliche Ratten reagieren auf PMSG (Pregnant Mare's Serum Gonadotropin)-Behandlung mit einheitlich erhöhter Steroidsynthese. Die Aromataseaktivität kann durch die Beeinflussung der PMSG-stimulierten Östrogenbildung gemessen werden.

Im sogenannten PMSG-Test werden 21 Tage alte weibliche Ratten alle 2 Tage insgesamt 7 mal mit je 100 I. U. PMSG subkutan vorbehandelt. 1 Stunde vor und 8 Stunden nach der letzten PMSG-Applikation ($d_{12}$) erhalten die Tiere die Testsubstanz in 0,1 ml Benzylbenzoat/Rizinusöl (1 + 9) durch subkutane Injektion. Die Kontrolltiere erhalten nur das Vehikel. 24 Studen nach der letzten PMSG-Applikation werden die Tiere getötet. Im Serum wird Östradiol radioimmunologisch geprüft. Für jede Gruppe von 10 Tieren wird der Mittelwert der Östradiolkonzentration in nMol/l mit der Standardabweichung errechnet. Die Signifikanzen der Unterschiede zur Kontrollgruppe werden durch die Varianzanalyse geprüft.

Zur Ermittlung der relativen Wirkungsstärke der zu testenden Substanz im Vergleich zu der Standardsubstanz wird die Regressions- und Kovarianzanalyse durchgeführt. Ferner wird die prozentuale Hemmung gegen die PMSG-Kontrolle berechnet.

Am Beispiel der erfindungsgemäßen Verbindung 15α (Acetoxyethyl)-1-methyl-androsta-1,4-dien-3,17-dion (B) wird gezeigt, daß die erfindungsgemäßen Verbindungen die Östradiolkonzentration im Serum weit stärker senken als das bekannte 4-Hydroxy-4-androsten-3,17-dion (A). Während eine Hemmung der Östradiol-Konzentration bei den erfindungsgemäßen Verbindungen schon mit 2 × 0,1 mg beobachtet wird, tritt eine solche Hemmung bei der Vergleichssubstanz erst mit 2 × 1,0 mg ein.

Tabelle

Beeinflussung der Östradiol-Konzentration im peripheren Serum bei der PMSG-vorbehandelten Ratte

| | Dosis mg/Tier 2 x s.c. | n | Östradiol-Konzentration nMol/l | Hemmung % |
|---|---|---|---|---|
| PMSG-Kontrolle | - | 10 | 3,42 ± 0,56 | - |
| A | 0,1 | 10 | 3,98 ± 1,12 | - |
| | 0,3 | 10 | 3,84 ± 0,84 | - |
| | 1,0 | 10 | 1,94 ± 0,62 | 44 |
| | 3,0 | 10 | 0,98 ± 0,19 | 71 |
| B | 0,1 | 10 | 2,11 ± 0,39 | 38 |
| | 0,3 | 10 | 0,62 ± 0,13 | 82 |
| | 1,0 | 10 | 0,54 ± 0,07 | 84 |
| | 3,0 | 10 | 0,36 ± 0,06 | 89 |

n = Tierzahl pro Gruppe

In einem weiteren Test wird der Einfluß von Aromatasehemmern auf den Östruszyklus der Ratte bestimmt.

Dies wird am Beispiel der erfindungsgemäßen Verbindung 15α-(1S-Butyryloxy-ethyl)-1-methyl-androsta-1,4-dien-3,17-dion (C) gezeigt :

Die Substanzen, die die Östrogenbiosynthese bzw. die Wirkung von Östrogenen hemmen, führen zu einer Störung des Östruszyklus bei Nagern.

Verwendet werden weibliche Ratten (SPF-Wistar) von ca. 180 g Körpergewicht. Pro Gruppe werden 10 Tiere eigesetzt. Der Zyklus wird mittels der Allen-Doisy-Abstrichtechnik täglich untersucht. Es werden zwei aufeinanderfolgende Zyklen verfolgt. Nur Tiere mit einem regelmäßigen 4-Tage-Zyklus kommen in den Versuch. Am Tage des Metöstrus werden die Tiere einmal mit der Testsubstanz (20 mg/Tier) subcutan behandelt. Die Testsubstanz wird dabei in einer 0,9 %igen Kochsalzlösung mit Myrj-53(R) (0,085 %) suspendiert (0,5 ml/Dosis). Die Kontrolltiere werden nicht behandelt. Der Versuch wird beendet, wenn alle behandelten Tiere wieder normale Zyklen aufweisen.

Alle unbehandelten Tiere zeigen während des 30 tägigen Versuchs einen regelmäßigen 4-Tage-Zyklus. Die einmalige subcutane Behandlung der Tiere mit 20 mg der erfindungsgemäßen Verbindung (C)/Tier führt zu einer Störung des Zyklus. Die Wirkung tritt gleich nach der Behandlung auf : die meisten Tiere zeigen dabei einen 5-Tage-Zyklus. Diese Störung des Zyklus dauert etwa 9 bis 27 Tage (Durchschnitt : etwa 15 Tage). Danach zeigen die behandelten Tiere wieder normale 4-Tage-Zyklen.

Die neuen Verbindungen der allgemeinen Formel I sind als Aromatasehemmer zur Hemmung der Östrogenbiosynthese und damit zur Behandlung von Krankheiten geeignet, die durch Östrogene bedingt oder von Östrogenen abhängig sind. So sind die neuen Verbindungen geeignet zur Behandlung von östrogeninduzierten oder -stimulierten Tumoren, wie zum Beispiel Mammakarzinom oder Prostatahyperplasie.

Die neuen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.

Wahrscheinlich sind Aromatasehemmer auch zur Behandlung des drohenden Herzinfarkts geeignet, da erhöhte Östrogenspiegel beim Mann einem Herzinfarkt vorangehen.

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01-100 mg/kg Körpergewicht, vorzugsweise 0,1-20 mg/kg Körpergewicht, je Tag betragen.

Zur oralen Verabreichung kommen Kapseln, Pillen, Tabletten, Dragées usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierung seinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind zum Beispiel Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogen bedingten Krankheiten.

Die neuen Verbindungen der allgemeinen Formel I werden dadurch erhalten, dass man das 17-Hydroxysteroid der allgemeinen Formel II

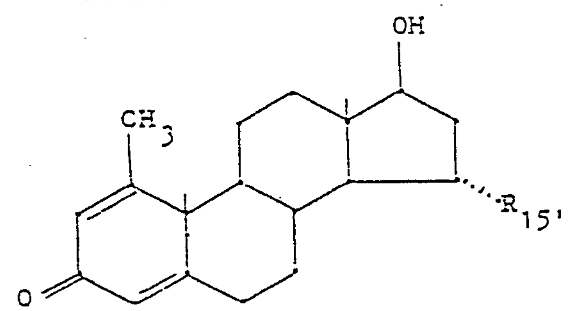

(II)

worin $R_{15'}$ eine Alkylgruppe mit 1 bis 4 Kohlenstoffatomen darstellt, die gegebenenfalls in 1- und/oder 2-Stellung durch eine oder zwei geschützte Hydroxygruppen substituiert ist, oxydiert, gegebenenfalls Schutzgruppen abspaltet und gegebenenfalls Monohydroxygruppen alkanoyliert oder Dihydroxygruppen mit Aceton umsetzt.

Die Oxydation kann in an sich bekannter Weise zum Beispiel mit Chromsäurereagenzien (Jones'-Reagenz oder Chromsäure-Pyridin) oder mit Pyridiniumdichromat oder -chlorchromat durchgeführt werden.

Die Abspaltung der Hydroxyschutzgruppen geschieht ebenfalls in an sich bekannter Weise. Als Schutzgruppen von Monohydroxygruppen kommen insbesondere Alkanoylgruppen mit 2 bis 7 Kohlenstoffatomen infrage. Alkanoylgruppen können mit einer anorganischen Base in alkoholischer Lösung verseift werden. Die sich gegebenenfalls anschließende Veresterung wird vorzugsweise mit dem entsprechenden Säureanhydrid oder -halogenid in Gegenwart von organischen Basen vorgenommen.

Als Schutzgruppe von 1,2-Dihydroxygruppen ist besonders die Isopropylidengruppe zu nennen. Die Isopropylidengruppe kann mit anorganischen Säuren in wäßrigen organischen Lösungsmitteln entfernt werden.

Das Ausgangsmaterial der allgemeinen Formel II wird über 17β-Hydroxy-1α-methyl-3α-tetrahydropyranyloxy-5α-androstan-15α-carbaldehyd hergestellt :

Die Herstellung des 15α-Carbaldehyds sei anhand des folgenden Schemas erläutert :

Schema

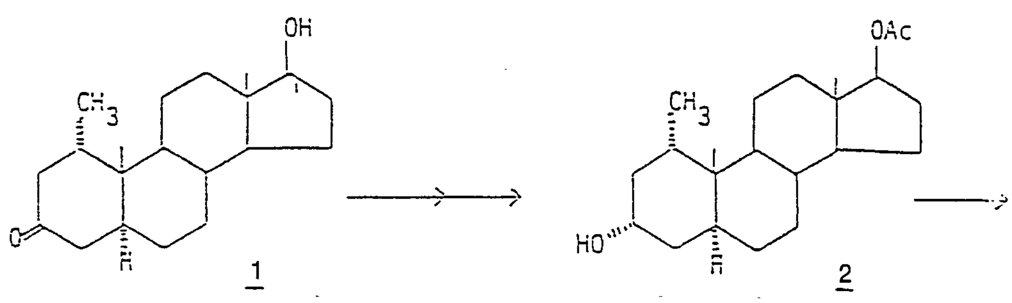

1

2

F 135.5-136 °C

Schema (Fortsetzung)

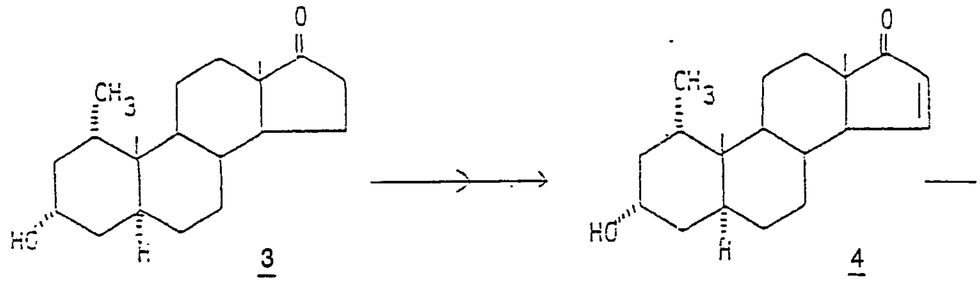

3

4

F 151.5-152 °C

F 167-168 °C

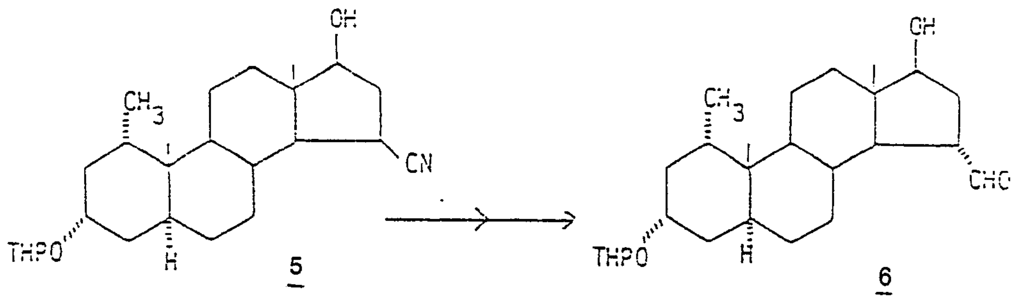

5

6

Herstellung des 17β-Hydroxy-1α-methyl-3α-tetrahydropyranyloxy-5α-androstan-15α-carbaldehyds der Formel 6

Ausgehend vom 1α-Methyl-17β-hydroxy-5α-androstan-3-on (1) wird nach Acetylierung und Reduktion mit Lithiumaluminium-tri-tert.-butoxyhydrid 2 erhalten. Veretherung der 3-Hydroxygruppe, Verseifung des 17-Acetats, Oxidation mit Pyridiniumdichromat und Spaltung der Schutzgruppe ergibt 3. Nach Ketalisierung, Bromierung, Bromwasserstoffabspaltung und Hydrolyse des Ketals wird 4 gewonnen. Veretherung, Addition von Blausäure und Reduktion der Ketogruppen führt zu 5, das nach Isomerisierung und Dibah-Reduktion die Schüsselverbindung 6 ergibt.

Herstellung des 17β-Hydroxy-1-methyl-15α-alkyl-androsta-1,4-dien-3-ons der allgemeinen Formel II

Die Herstellung des 17β-Hydroxy-Steroids der allgemeinen Formel II ist dadurch gekennzeichnet, daß man im 17β-Hydroxy-1α-methyl-3α-tetrahydropyranyloxy-5α-androstan-15α-carbaldehyd der Formel 6

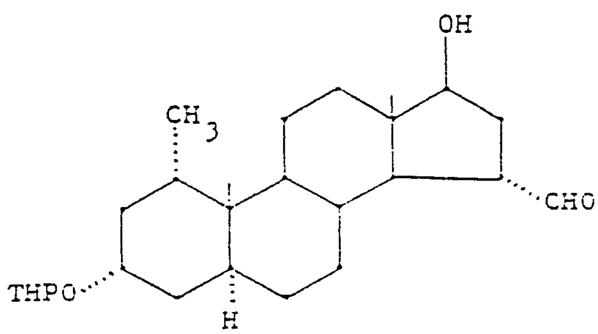

6

die 15-Formylgruppe — gegebenenfalls nach einer Wittig- oder Corey-Reaktion — reduziert oder das nach der Wittig-Reaktion entstandene 15-Olefin durch Epoxidierung und anschließende Öffnung des Epoxids oder durch Umsetzung mit N-Morpholin-N-oxid in Gegenwart von Osmiumtetroxid hydroxyliert, die 17-Hydroxygruppe und im 15α-Alkylrest vorhandene Hydroxygruppen schützt, den 3-Tetrahydropyranylether spaltet, wobei die Schutzgruppeneinführung und die 3-Etherspaltung auch unmittelbar im Anschluß an die Wittig-Reaktion durchgeführt werden können, die 3-Hydroxygruppe oxydiert, durch Bromierung und Bromwasserstoffabspaltung Doppelbindungen in 1,2- und 4,5-Stellung einführt und die 17-O-Schutzgruppe abspaltet.

Die im Falle einer Hydroxy- bzw. Alkanoyloxy-Substitution der $R_{15}$-Alkylgruppe auftretenden R/S-Gemische können gewünschtenfalls auf chromatographischem Wege getrennt werden.

Beispiel 1

a) Zu 4,29 g Triphenylmethylphosphoniumbromid in 40 ml Dimethylsulfoxid werden unter Argon 1,35 g Kalium-tert-butylat eingetragen. In diese Ylid-Lösung werden 1,67 g 17β-Hydroxyl-1α-methyl-3α-tetrahydropyranyloxy-5α-androstan-15α-carbaldehyd zugegeben, anschließend wird 5 Minuten bei Raumtemperatur gerührt. Danach wird in Eis-Natriumchlorid-Lösung gefällt, mit Methylenchlorid extrahiert und an Silicagel chromatographiert. Durch Elution mit Methylenchlorid-Essigester 85 : 15 werden 1,53 g 15α-Ethenyl-17β-hydroxy-1α-methyl-3α-tetrahydropyranyloxy-5α-androstan vom Schmelzpunkt 87-93 °C erhalten.

b) 1,7 g 15α-Ethenyl-17β-hydroxy-1α-methyl-3α-tetrahydropyranyloxy-5α-androstan werden in 3 ml Pyridin mit 1,5 ml Acetanhydrid 30 Minuten auf dem Dampfbad erhitzt, in Eis-Wasser gefällt, abgesaugt und mit Wasser gewaschen. Das rohe 17-Acetat wird in 20 ml Methanol, 5 ml Wasser und 1,0 g Oxalsäure 50 Minuten am Rückfluß erhitzt. Nach Eis-Wasser-Fällung wird das Produkt mit Methylenchlorid extrahiert und eingedampft. Durch Chromatographie an Silicagel erhält man 1,34 g amorphes 17β-Acetoxy-15α-ethenyl-3α-hydroxy-1α-methyl-5α-androstan.

c) 3,65 g 17β-Acetoxy-15α-ethenyl-3α-hydroxy-1α-methyl-5α-androstan werden in 90 ml Methanol mit 180 mg 10 %iger Palladium-Kohle hydriert. Der Katalysator wird abfiltriert, die Lösung eingedampft und aus Methanol-Wasser kristallisiert. Man erhält 3,5 g 17β-Acetoxy-15α-ethyl-3α-hydroxy-1α-methyl-5α-androstan vom Schmelzpunkt 65-73 °C.

d) 3,5 g 17β-Acetoxy-15α-ethyl-3α-hydroxy-1α-methyl-5α-androstan werden in 60 ml Aceton mit 12 ml einer Chromsäure-Lösung (hergestellt aus 6,67 g $CrO_3$, 90 ml $H_2O$ und 6 ml konz. $H_2SO_4$) tropfenweise versetzt. Nach Fällung in Eis-Wasser, Absaugen, Waschen mit Wasser und Trocknen erhält man 3,38 g 17β-Acetoxy-15α-ethyl-1α-methyl-5α-androstan-3-on.

e) 3,37 g 17β-Acetoxy-15α-ethyl-1α-methyl-5α-androstan-3-on werden in 34 ml Eisessig gelöst und unter Kühlung auf 15 °C eine Lösung von 1,01 ml Brom in 15 ml Eisessig innerhalb von 15 Minuten zugetropft und 10 Minuten nachgerührt. Nach Fällung in Eis-Wasser wird abgesaugt, neutralgewaschen und im Vakuum getrocknet. 4,8 g rohe 2,4-Dibrom-Verbindung werden in 48 ml Dimethylformamid mit 4,8 g Lithiumcarbonat versetzt und 5 Stunden bei 120 °C Badtemperatur gerührt. Nach Wasserfällung, Absaugen und Waschen mit Wasser wird das rohe 1,4-Dien mit 20 ml 3 %iger Kaliumhydroxid-Lösung in Methanol verseift und aufgearbeitet.

f) 2,01 g nach e) erhaltenes 15α-Ethyl-17β-hydroxy-1-methyl-androsta-1,4-dien-3-on werden in 40 ml Aceton mit 7 ml Chromsäure-Lösung oxidiert und isoliert wie unter d) beschrieben. Durch Umkristallisation aus Aceton-Hexan erhält man 1,8 g 15α-Ethyl-1-methyl-androsta-1,4-dien-3,17-dion vom Schmelzpunkt 161,5-162 °C.

Beispiel 2

Analog Beispiel 1 a)-f) wird mit Ethyltriphenylphosphoniumbromid das 15α-Propyl-1-methyl-androsta-1,4-dien-3,17-dion hergestellt.

Schmelzpunkt 189-190,5 °C.

Beispiel 3

a) 6,0 g. 17β-Hydroxy-1α-methyl-3α-tetrahydropyranyloxy-5α-androstan-15α-carbaldehyd werden in 60 ml Dimethylformamid gelöst, 5,86 g Trimethylsulfoniumjodid werden hinzugefügt und es wird im Eisbad gekühlt. Danach werden 4,15 g Kalium-tert-butylat eingetragen und bei 5 °C 25 Minuten unter Argon gerührt. Die Reaktionslösung wird in Eiswasser gegossen, die Fällung abgesaugt, in Methylenchlorid gelöst, eingedampft und an Silicagel chromatographiert. Man erhält so 5,13 g 17β-Hydroxy-1α-methyl-15α-(2S-oxiranyl)-3α-tetrahydropyranyloxy-5α-androstan (Schmelzpunkt 133-150 °C) und 610 mg 17β-Hydroxy-1α-methyl-15α(2R-oxiranyl)-3α-tetrahydropyranyloxy-5α-androstan (Schmelzpunkt 158-173 °C).

b) 5,1 g 17β-Hydroxy-1α-methyl-15α-(2 S-oxiranyl)3α-tetrahydropyranyloxy-5α-androstan werden in 100 ml Tetrahydrofuran gelöst, im Eisbad gekühlt und 1,8 g Lithium-aluminiumhydrid portionsweise eingetragen. Es wird 2 Stunden bei Raumtemperatur nachgerührt und mit Ammoniumchlorid-Lösung zerlegt. Nach Extraktion mit Essigester, Waschen mit gesättigter Natriumchlorid-Lösung und Eindampfen wird der Rückstand aus Aceton-Hexan umkristallisiert. Man erhält 4,63 g 15α(1S-hydroxyethyl)-17β-hydroxy-1α-methyl-3α-tetrahydropyranyloxy-5α-androstan vom Schmelzpunkt 193-195 °C.

c) 4,6 g 15α(1S-Hydroxyethyl)-17β-hydroxy-1α-methyl-3α-tetrahydropyranyloxy-5α-androstan werden in 18 ml Pyridin und 9 ml Acetanhydrid 30 Minuten auf dem Dampfbad erhitzt. Es wird in Eis-Wasser gefällt, abgesaugt und gewaschen. Das Rohprodukt wird in 60 ml Methanol mit 2,0 g Oxalsäure in 30 ml Wasser versetzt und 10 Minuten am Rückfluß erhitzt und in Eis-Wasser gefällt und aufgearbeitet. 4,3 g Rohprodukt werden in 40 ml Aceton mit 3,15 ml Jones'-Reagenz 10 Minuten im Eisbad gerührt, anschließend in Wasser gefällt, aufgearbeitet und an Silicagel chromatographiert. Nach Kristallisation

aus Ether-Pentan erhält man 4,1 g 15α(1S-Acetoxyethyl)-17β-acetoxy-1α-methyl-5α-androstan-3-on vom Schmelzpunkt 118-120 °C.

d) 4,1 g 15α(1S-Acetoxyethyl)-17β-acetoxy-1α-methyl-5α-androstan-3-on werden bromiert und Bromwasserstoff abgespalten wie im Beispiel 1 e) beschrieben. Man erhält nach Umkristallisation aus Ether-Pentan 2,3 g 15α(1S-Acetoxyethyl)-17β-acetoxy-1-methyl-androsta-1,4-dien-3-on vom Schmelzpunkt 152-154 °C.

e) 1,67 g 15α(1S-Acetoxyethyl)-17β-acetoxy-1-methyl-androsta-1,4-dien-3-on werden in 30 ml 0,5 %iger methanolischer Kaliumhydroxid-Lösung 4,5 Stunden bei Raumtemperatur gerührt, mit Essigsäure neutralisiert, eingeengt und chromatographiert. Nach Umkristallisation aus Aceton-Hexan erhält man 1,24 g 15α(1S-Acetoxyethyl)-17β-hydroxy-1-methyl-androsta-1,4-dien-3-on vom Schmelzpunkt 206,5-208,5 °C.

f) 950 mg 15α(1S-Acetoxyethyl)-17β-hydroxy-1-methyl-androsta-1,4-dien-3-on werden in 25 ml Aceton mit 0,75 ml Jones'-Reagenz 10 Minuten im Eisbad gerührt. Es wird in Eis-Wasser gefällt, abgesaugt, mit Wasser gewaschen, getrocknet und aus Aceton-Hexan umkristallisiert. Man erhält 920 mg 15α(1S-Acetoxyethyl)-1-methylandrosta-1,4-dien-3,17-dion vom Schmelzpunkt 196,5-197 °C.

Beispiel 4

440 mg des nach Beispiel 3 erhaltenen 15α(1S-Acetoxyethyl)-1-methyl-androsta-1,4-dien-3,17-dions werden in 10 ml 3 %iger methanolischer Kaliumhydroxid-Lösung 3,5 Stunden unter Argon gerührt, mit Essigsäure neutralisiert, aufgearbeitet und aus Aceton-Hexan umkristallisiert. Man erhält 350 mg 15α(1S-Hydroxyethyl)-1-methyl-androsta-1,4-dien-3,17-dion vom Schmelzpunkt 243-244,5 °C.

Beispiel 5

a) 8,4 g 17β-Hydroxy-1α-methyl-3α-tetrahydropyranyloxy-5α-androstan-15α-carbaldehyd werden in 100 ml Tetrahydrofuran mit 7,6 g Lithiumaluminium-tri-tert-butoxyhydrid 1 Stunde bei Raumtemperatur gerührt. Anschließend wird in 500 ml Eis-Wasser — dem 40,0 g Weinsäure zugesetzt war — gefällt, das Produkt abgesaugt, mit Wasser gewaschen und getrocknet. Man erhält 8,5 g 15α-Hydroxymethyl-17β-hydroxy-1α-methyl-3α-tetrahydropyranyloxy-3α-androstan, die in 30 ml Pyridin und 15 ml Acetanhydrid 2,5 Tage bei 20 °C gerührt werden. Nach Eis-Wasser-Fällung, Absaugen und Waschen wird das Rohprodukt in 80 ml Methanol gelöst, mit 3,0 g Oxalsäure in 35 ml Wasser versetzt und 15 Minuten am Rückfluß erhitzt. Nach Aufarbeitung, Chromatographie an Silicagel und Umkristallisation aus Methanol erhält man 6,5 g 15α-Acetoxymethyl-17β-acetoxy-3α-hydroxy-1α-methyl-5α-androstan vom Schmelzpunkt 67-68 °C.

b) 6,3 g 15α-Acetoxymethyl-17β-acetoxy-3α-hydroxy-1α-methyl-5α-androstan werden — wie im Beispiel 1 d) und 1 e) beschrieben — oxidiert, bromiert und Bromwasserstoff abgespalten. Man erhält so 3,5 g 15α-Acetoxy-methyl-17β-acetoxy-1-methyl-androsta-1,4-dien-3-on vom Schmelzpunkt 204-205 °C.

c) 3,5 g 15α-Acetoxymethyl-17β-acetoxy-1-methyl-androsta 1,4-dien-3-on werden mit 60 ml 2,5 %iger methanolischer Kaliumhydroxid-Lösung 2,5 Stunden bei 20 °C gerührt. Danach wird mit Essigsäure neutralisiert und aufgearbeitet. Man erhält nach Umkristallisation aus Aceton-Ether 2,8 g 15α-Hydroxymethyl-17β-hydroxy-1-methyl-androsta-1,4-dien-3-on vom Schmelzpunkt 183,5-185 °C.

d) 2,45 g 15α-Hydroxymethyl-17β-hydroxy-1-methyl-androsta-1,4-dien-3-on werden mit 20 ml Dimethylformamid, 650 mg Bleidiacetat und 6 ml Acetanhydrid 7,5 Stunden bei 20 °C gerührt. Es wird aufgearbeitet und aus Ether-Pentan umkristallisiert. Erhalten werden 2,17 g 15α-Acetoxymethyl-17β-hydroxy-1-methyl-androsta-1,4-dien-3-on vom Schmelzpunkt 198-200 °C.

e) 1,9 g 15α-Acetoxymethyl-17β-hydroxy-methyl-androsta-1,4-dien-3-on werden in 60 ml Aceton mit 1,7 ml Jones'-Reagenz oxidiert und aufgearbeitet. Nach Umkristallisation aus Aceton-Hexan erhält man 1,8 g 15α-Acetoxymethyl-1-methyl-androsta-1,4-dien-3,17-dion vom Schmelzpunkt 179-181 °C.

Beispiel 6

1,56 g 15α-Acetoxymethyl-1-methyl-androsta-1,4-dien-3,17-dion werden — wie im Beispiel 5 c) beschrieben — verseift. Nach Kristallisation aus Aceton erhält man 1,2 g 15α-Hydroxymethyl-1-methyl-androsta-1,4-dien-3,17-dion vom Schmelzpunkt 250-252 °C.

Beispiel 7

350 mg 15α-Hydroxymethyl-1-methyl-androsta-1,4-dien-3,17-dion werden in 1,5 ml Pyridin mit 0,35 ml Valeriansäureanhydrid 15 Minuten auf dem Dampfbad erhitzt, auf Raumtemperatur abgekühlt und gefällt. Nach Kristallisation aus Ether-Pentan erhält man 325 mg 1-Methyl-15α-valeryloxy-methyl-androsta-1,4-dien-3,17-dion vom Schmelzpunkt 92-95 °C.

Beispiel 8

a) Zu 23,12 g Propyltriphenylphosphoniumbromid in 150 ml Dimethylsulfoxid werden unter Argon-Begasung 6,74 g Kalium-tert-butylat eingetragen. Zu dieser Ylid-Lösung werden 8,38 g 17β-Hydroxy-1α-methyl-3α-tetrahydropyranyloxy-5α-androstan-15α-carbaldehyd in 40 ml Dimethylsulfoxid zugetropft und 10 Minuten bei Raumtemperatur nachgerührt. Nach Aufarbeitung — wie im Beispiel 1 a) beschrieben — werden 7,08 g 15α-Butenyl-17β-hydroxy-1α-methyl-3α-tetrahydropyranyloxy-5α-androstan erhalten.

b) Nach Acetylierung und Etherspaltung — wie im Beispiel 1 b) beschrieben — erhält man 4,52 g 17β-Acetoxy-15α-butenyl-3α-hydroxy-1α-methyl-5α-androstan vom Schmelzpunkt 133-134 °C.

c) Nach Hydrierung, Oxidation. Bromierung, Bromwasserstoffabspaltung, Verseifung und Oxidation analog Beispiel 1 c)-f) erhält man das 15α-Butyl-1-methyl-androsta-1,4-dien-3,17-dion vom Schmelzpunkt 161-162 °C.

Beispiel 9

4,4 g 17β-Hydroxy-1α-methyl-3α-tetrahydropyranyloxy-5α-androstan-15α-carbaldehyd werden in 25 ml Diethylenglykol gelöst, 5,3 ml Hydrazinhydroxid zugetropft und anschließend bei 130 °C Badtemperatur 80 Minuten erhitzt. Danach werden 4,4 g Kaliumhydroxid zugegeben und die Badtemperatur für 90 Minuten auf 225 °C gesteigert. Nach dem Abkühlen wird die Reaktionslösung in Eis-Wasser gefällt, das Produkt abgesaugt, gewaschen und getrocknet. Man erhält so 4,5 g 17β-Hydroxy-1α, 15α-dimethyl-3α-tetrahydropyranyloxy-5α-androstan, das nach Acetylierung der 17-Hydroxygruppe und Spaltung des 3-Tetrahydropyranylethers analog Beispiel 1 d)-f) umgesetzt wird. Nach Umkristallisation aus Aceton-Hexan werden 1,5 g 1,15α-Dimethyl-androsta-1,4-dien-3,17-dion vom Schmelzpunkt 153-155 °C erhalten.

Beispiel 10

200 mg 15α(1-Hydroxyethyl)-1-methyl-androsta-1,4-dien-3,17-dion werden mit 0,3 ml Buttersäureanhydrid in 1,5 ml Pyridin umgesetzt wie im Beispiel 7 beschrieben. Man erhält so 150 mg 15α(1-Butyryloxyethyl)-1-methyl-androsta-1,4-dien-3,17-dion vom Schmelzpunkt 82-84 °C.

Beispiel 11

a) 8,4 g 17β-Acetoxy-3α-hydroxy-1α-methyl-15α-prop-1-enyl-5α-androstan werden in 50 ml Tetrahydrofuran gelöst, mit 5 g N-Methylmorpholin-N-oxid, 15 ml Wasser und 20 ml t-Butanol versetzt und unter Rühren eine Lösung von 100 mg Osmiumtetroxid in 15 ml Tetrahydrofuran hinzugefügt. Anschließend wird in schwefelsaures Eis/Wasser, das mit 500 mg Natriumsulfid versetzt war, gefällt, das Produkt abgesaugt, mit Wasser gewaschen und im Vakuum getrocknet. Das Rohprodukt (8,6 g) wird in 100 ml Tetrahydrofuran und 100 ml Aceton mit 0,5 ml Bortrifluorid-Etherat versetzt und 30 Minuten bei 0 °C gerührt. Nach Zugabe von 1 ml Pyridin wird im Vakuum eingeengt, mit Wasser gefällt, abgesaugt, mit Wasser gewaschen und getrocknet. Nach Umkristallisation aus Aceton-Hexan erhält man 6,2 g 17β-Acetoxy-3α-hydroxy-1α-methyl-15α-(1,2-isopropylidendioxypropyl)-5α-androstan vom Schmelzpunkt 198-201 °C.

b) 6,8 g 17β-Acetoxy-3α-hydroxy-1α-methyl-15α(1,2-isopropylidendioxypropyl)-5α-androstan werden in 25 ml Dimethylformamid gelöst, in die Lösung werden 11 g Pyridiniumdichromat fest eingetragen, dabei steigt die Innentemperatur auf + 35 °C an. Es wird auf + 20 °C gekühlt und 3,5 Stunden gerührt. Anschließend wird in Eis/Wasser gefällt, das Produkt abgesaugt, mit Wasser gewaschen und im Trockenschrank getrocknet. Das rohe 17β-Acetoxy-1α-methyl-15α(1,2-isopropylidendioxypropyl)-5α-androstan-3-on wird bromiert (wobei die Schutzgruppe abspaltet) und dehydrobromiert wie im Beispiel 1 e) beschrieben. Man erhält nach Kristallisation aus Aceton 4,5 g 17β-Acetoxy-1-methyl-15α(1,2-dihydroxypropyl)-androsta-1,4-dien-3-on vom Schmelzpunkt 197-199 °C.

c) 4,2 g Diol werden in 50 ml Aceton und 50 ml Tetrahydrofuran mit 0,25 ml Bortrifluorid-Etherat versetzt und 30 Minuten bei 0 °C gerührt. Nach Aufarbeitung wird das rohe 17β-Acetoxy-1-methyl-15α-(1,2-isopropylidendioxypropyl)-androsta-1,4-dien-3-on mit 3 %iger Kaliumhydroxid-Lösung verseift und aufgearbeitet. Nach Oxidation mit Pyridiniumdichromat, wie unter Beispiel 11 b) beschrieben, wird aus Aceton-Hexan kristallisiert. Man erhält 2,8 g 1-Methyl-15α(1,2-isopropylidendioxypropyl)-androsta-1,4-dien-3,17-dion vom Schmelzpunkt 162-164 °C.

Beispiel 12

1 g 1-Methyl-15α(1,2-isopropylidendioxypropyl)-androsta-1,4-dien-3,17-dion werden in 100 ml 10 %ig wäßrigen Tetrahydrofuran mit 2 ml 0,1 m Salzsäure versetzt, 2 Stunden bei 20 °C gerührt und aufgearbeitet. Nach Umkristallisation aus Aceton erhält man 650 mg 1-Methyl-15α(1,2-dihydroxypropyl)-androsta-1,4-dien-3,17-dion vom Schmelzpunkt 177-179 °C.

Beispiel 13

a) 24,5 g 17β-Acetoxy-15α-but-1-en-yl-3α-hydroxy-1α-methyl-5α-androstan werden in 500 ml Ethylenchlorid gelöst, 23 g p-Nitroperbenzoesäure zugesetzt und 25 Minuten bei Raumtemperatur gerührt. Nach Verdünnen mit Methylenchlorid wird mit 1 M Natriumhydroxid-Lösung und mit Wasser gewaschen und im Vakuum eingedampft.

b) Man erhält 22,8 g 17β-Acetoxy-15α(1,2-epoxybutyl)-3α-hydroxy-1α-methyl-5α-androstan, die in 200 ml Toluol und 20 ml Dihydropyran mit 100 mg p-Toluolsulfonsäure 1 Stunde bei Raumtemperatur gerührt werden. Nach Zugabe von 1 ml Pyridin wird aufgearbeitet. Man erhält so 23,8 g 17β-Acetoxy-15α(1,2-epoxybutyl)-1α-methyl-3α-tetrahydropyranyloxy-5α-androstan.

c) 23,5 g 17β-Acetoxy-15α(1,2-epoxybutyl)-1α-methyl-3α-tetrahydropyranyloxy-5α-androstan werden in 400 ml Tetrahydrofuran mit 23,5 g Lithiumaluminiumhydrid 1 Stunde am Rückfluß erhitzt. Es wird auf — 20 °C gekühlt und 100 ml Essigester zugetropft. Anschließend wird mit Essigester verdünnt und mit verdünnter Schwefelsäure, Natriumhydrogencarbonat und Wasser gewaschen und die organische Phase eingedampft. Der Rückstand wird wie im Beispiel 3 c) beschrieben acetyliert und der Tetrahydropyranylether gespalten. Nach Chromatographie an Silicagel, Gradienten-Elution mit Methylenchlorid-Aceton 9 : 1, erhält man 5,2 g 17β-Acetoxy-15α(1-acetoxybutyl)-3α-hydroxy-1α-methyl-5α-androstan vom Schmelzpunkt 122-125 °C und 7,8 g 17β-Acetoxy-15α(2-Acetoxybutyl)-3α-hydroxy-1α-methyl-5α-androstan vom Schmelzpunkt 137-139 °C.

d) Beide 17β-Acetoxybutyl-Verbindungen werden getrennt oxidiert, bromiert, dehydrobromiert, verseift und oxidiert wie im Beispiel 3 c)-f) beschrieben. Man erhält so : 15α(1-Acetoxybutyl)-1-methyl-androsta-1,4-dien-3,17-dion vom Schmelzpunkt 173-175 °C und 15α(2-Acetoxybutyl)-1-methyl-androsta-1,4-dien-3,17-dion vom Schmelzpunkt 151-152,5 °C.

Beispiel 14

550 mg des nach Beispiel 4 erhaltenen 15α-(1S-Hydroxyethyl)-1-methyl-androsta-1,4-dien-3,17-dions werden mit 0,55 ml Valeriansäureanhydrid, 50 mg 4-Dimethylaminopyridin in 3 ml Pyridin 30 Minuten auf dem Dampfbad erhitzt. Nach Aufarbeitung, wie im Beispiel 7 beschrieben, erfolgt Umkristallisation aus Aceton-Hexan. Man erhält 600 mg 1-Methyl-15α-(1S-valeryloxyethyl)-androsta-1,4-dien-3,17 dion vom Schmelzpunkt 174-175 °C.

Beispiel 15

a) Zu 102,5 g Ethyltriphenylphosphoniumbromid in 500 ml Dimethyl sulfoxid werden unter Argon-Begasung 31 g Kalium-tert-butylat eingetragen. Zu dieser Ylid-Lösung gibt man unter Rühren 38,5 g 17β-Hydroxy-1α-methyl-3α-tetrahydropyranyloxy-5α-androstan-15α-carbaldehyd. Nach einer Reaktionszeit von 10 Minuten wird in Eiswasser gefällt, mit Essigsäure neutralisiert und abgesaugt. Das Produkt wird in Methylenchlorid gelöst und an Silicagel chromatographiert. Man erhält so 36,5 g 15α-Propenyl-17β-hydroxy-1α-methyl-3α-tetrahydropyranyloxy-5α-androstan, die analog Beispiel 1b zum 17β-Acetoxy-3α-hydroxy-1α-methyl-15α-propenyl-5α-androstan umgesetzt werden. Nach Kristallisation aus Aceton-Hexan erhält man 30,6 g obiger Verbindung vom Schmelzpunkt 172-173 °C.

b) 970 mg 17β-Acetoxy-3α-hydroxy-1α-methyl-15α-propenyl-5α-androstan werden in 80 ml Ethylenchlorid gelöst, mit 1,1 g p-Nitroperbenzoesäure versetzt und 1 Stunde bei 0-5 °C gerührt. Nach Verdünnen mit Methylenchlorid wird mit 1 M Natriumhydroxid-Lösung und mit Wasser gewaschen und im Vakuum eingedampft. Man erhält nach Umkristallisation aus Pentan 740 mg 17β-Acetoxy-15α-(1,2-epoxypropyl)-3α-hydroxy-1α-methyl-5α-androstan vom Schmelzpunkt 139-140 °C.

c) 370 mg 17β-Acetoxy-15α-(1,2-epoxypropyl)-3α-hydroxy-1α-methyl 5α-androstan werden in 6 ml Methylenchlorid mit 650 mg Pyridiniumdichromat 5 Stunden bei 22 °C gerührt und über Silicagel filtriert. Die Lösung wird eingedampft und aus Ether-Pentan umkristallisiert. Man erhält so 360 mg 17β-Acetoxy-15α-(1,2-epoxypropyl)-1α-methyl-5α-androstan-3-on vom Schmelzpunkt 150-152 °C.

d) 370 mg 17β-Acetoxy-15α-(1,2-epoxypropyl)-1α-methyl-5α-androstan-3-on werden in 6 ml Eisessig mit 740 mg Lithiumbromid versetzt und 20 Minuten bei Raumtemperatur gerührt. Nach Fällung in Eiswasser, Absaugen und Trocknen wird das Rohprodukt an Silicagel chromatographiert und aus Aceton-Hexan umkristallisiert. Man erhält so 354 mg 17β-Acetoxy-15α-(2R-brom-1S-hydroxy-propyl)-1α-methyl-5α-androstan-3-on vom Schmelzpunkt 207,5-208,5 °C (Zersetzung).

332 mg obiger Verbindung werden in 5 ml Toluol mit 1 ml Tributylzinnhydrid und 40 mg Azoisobutyronitril 10 Minuten bei 50 °C gerührt. Zur Aufarbeitung wird mit Essigester verdünnt, mit Wasser gewaschen, das Lösungsmittel abgezogen und chromatographiert. Man erhält so 225 mg 17β-Acetoxy-15α-(1S-hydroxypropyl)-1α-methyl-5α-androstan-3-on vom Schmelzpunkt 153-154 °C.

214 mg obiger Verbindung werden in 1 ml Pyridin mit 0.5 ml Acetanhydrid umgesetzt. Nach Aufarbeitung und Kristallisation aus Aceton-Hexan erhält man 243 mg 17β-Acetoxy-15α-(1S-Acetoxypropyl)-1α-methyl-5α-androstan-3-on vom Schmelzpunkt 130-131 °C.

e) 9,4 g 17β-Acetoxy-15α-(1S-acetoxypropyl)-1α-methyl-5α-androstan-3-on werden bromiert und

Bromwasserstoff abgespalten, wie im Beispiel 1e beschrieben. Man erhält so 5,6 g 17β-Acetoxy-15α(1S-acetoxypropyl)-1α-methyl-androsta-1,4-dien-3-on vom Schmelzpunkt 172-173 °C.

f) 2,0 g 17β-Acetoxy-15α-(1S-acetoxypropyl)-1-methyl-androsta-1,4-dien-3-on werden verseift und oxidiert, wie in Beispiel 3e-f beschrieben. Nach Umkristallisation aus Aceton-Hexan erhält man 1,3 g 15α-(1S-Acetoxypropyl)-1-methyl-androsta-1,4-dien-3,17-dion vom Schmelzpunkt 210-211 °C.

Beispiel 16

a) 1,12 g 17β-Acetoxy-15α-(1,2-epoxybutyl)-3α-hydroxy-1α-methyl-5α-androstan werden in 5 ml Dimethylformamid mit 1,51 g Pyridiniumdichromat 1 Stunde bei Raumtemperatur oxidiert. Es wird in Eiswasser gefällt, das Produkt abgesaugt, getrocknet und aus Aceton-Hexan umkristallisiert. Man erhält so 720 mg 17β-Acetoxy-15α-(1,2-epoxybutyl)-1α-methyl-5α-androstan-3-on vom Schmelzpunkt 106-108 °C.

b) 630 mg obigen Epoxids werden in 10 ml Eisessig mit 1,2 g Lithiumbromid 20 Minuten bei Raumtemperatur gerührt, aufgearbeitet und an Silicagel chromatographiert. Man erhält so 535 mg 17β-Acetoxy-15α-(2R-brom-1S-hydroxybutyl)-1α-methyl-5α-androstan-3-on vom Schmelzpunkt 180-181 °C.

c) 500 mg des obigen Bromhydrins werden in 10 ml Toluol nach Zugabe von 50 mg Azoisobutyrolnitril und 1,5 ml Tributylzinnhydrid 45 Minuten bei 50 °C Badtemperatur gerührt. Nach Aufarbeitung und Chromatographie werden 400 mg 17β-Acetoxy-15α-(1S-hydroxybutyl)1α-methyl-5α-androstan-3-on mit Acetanhydrid und Pyridin umgesetzt, aufgearbeitet und chromatographiert. Man erhält so nach Umkristallisation aus Pentan 360 mg 17β-Acetoxy-15α-(1S-acetoxybutyl)-1α-methyl-5α-androstan-3-on vom Schmelzpunkt 106-107 °C.

d) 3,0 g 17β-Acetoxy-15α-(1S-acetoxybutyl)-1α-methyl-5α-androstan-3-on werden bromiert und Bromwasserstoff abgespalten, wie im Beispiel 1e beschrieben. Nach partieller Verseifung der 17β-Acetoxygrupe mit 0,5 %iger Kaliumhydroxid-Lösung in Methanol-Methylenchlorid und Umkristallisation aus Aceton-Hexan erhält man 620 mg 17β-Hydroxy-15α-(1S-acetoxybutyl)-1-methyl-1,4-androstadien-3-on vom Schmelzpunkt 217-220 °C.

e) 600 mg 17β-Hydroxy-15α-(1S-acetoxybutyl)-1-methyl-1,4-androstadien-3-on werden oxidiert, wie im Beispiel 1d beschrieben. Nach Kristallisation aus Aceton-Hexan erhält man 490 mg 15α-(1S-acetoxybutyl)-1-methyl-1,4-androstadien-3,17-dion vom Schmelzpunkt 186-187 °C.

Beispiel 17

a) 1,0 g 17β-Acetoxy-3α-hydroxy-1α-methyl-15α-(prop-1-enyl)-5α-androstan werden in 20 ml Methylenchlorid in 2,0 g Pyridiniumdichromat 5,5 Stunden bei Raumtemperatur gerührt. Anschließend wird mit Methylenchlorid verdünnt, über Silicagel filtriert und die Lösung eingedampft. Man erhält nach Umkristallisation aus Aceton-Hexan 738 mg 17β-Acetoxy-1α-methyl-15α-(prop-1-enyl)-5α-androstan-3-on vom Schmelzpunkt 129-130 °C.

b) 696 mg obigen Ketons werden in 5 ml Tetrahydrofuran gelöst, mit 435 mg N-Methylmorpholin-N-oxid, 1 ml Wasser und 1,8 ml t-Butanol versetzt und unter Rühren eine Lösung von 10 mg Osmiumtetroxid in 5 ml Tetrahydrofuran hinzugefügt. Nach einer Reaktionszeit von 16 Stunden wird aufgearbeitet, wie im Beispiel 11a beschrieben. Der Rückstand wird mit Pyridin und Acetanhydrid acetyliert. Nach Chromatographie und Umkristallisation aus Aceton-Hexan erhält man 730 mg 17β-Acetoxy-15α-(1R, 2S-diacetoxypropyl)1α-methyl-5α-androstan-3-on vom Schmelzpunkt 166,5-167,5 °C. Nach Bromierung, Dehydrobromierung und alkalischer Hydrolyse wird, wie in den Beispielen 11c und 12 beschrieben, mit Aceton umgesetzt, oxidiert und das Acetonid gespalten, so daß man 1-Methyl-15α-(1,2-dihydroxypropyl)-androsta-1,4-dien-3,17-dion vom Schmelzpunkt 177-179 °C erhält.

Beispiel 18

Das nach Beispiel 3a erhaltene 17β-Hydroxy-1α-methyl-15α-(2R-oxiranyl)-3α-tetrahydropyranyloxy-5α-androstan wird umgesetzt, wie im Beispiel 3b-f beschrieben. Man erhält nach Umkristallisation aus Essigester das 15α-(1R-Acetoxyethyl)-1-methyl-androsta-1,4-dien-3,17-dion vom Schmelzpunkt 228,5-229,5 °C.

Beispiel 19

200 mg des nach Beispiel 4 erhaltenen 15α-(1S-Hydroxyethyl)-1-methylandrosta-1,4-dions werden mit 0,3 ml Buttersäureanhydrid in 1,5 ml Pyridin analog Beispiel 7, umgesetzt. Man erhält 145 mg 15α-(1S-Butyryloxy-ethyl)-1-methyl-androsta-1,4-dien-3,17-dion vom Schmelzpunkt 192-193 °C.

**Patentansprüche**

1. 1-Methyl-15α-alkyl-androsta-1,4-dien-3,17-dione der allgemeinen Formel I

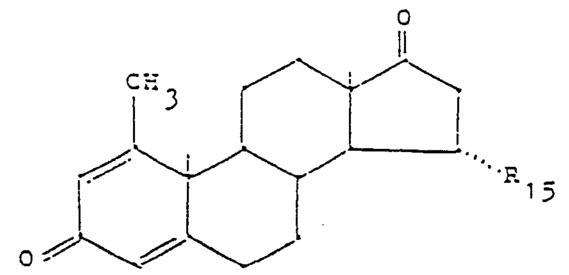

(I)

worin $R_{15}$ eine Alkylgruppe mit 1 bis 4 Kohlenstoffatomen darstellt, die gegebenenfalls in 1- und/oder 2-Stellung durch eine oder zwei Hydroxy- oder $C_2$-$C_7$-Alkanoyloxygruppen oder eine 1,2-Isopropylidendioxygruppe substituiert ist.

2. 15α-Ethyl-1-methyl-androsta-1,4-dien-3,17-dion,
1-Methyl-15α-propyl-androsta-1,4-dien-3,17-dion und
15α-Butyl-1-methyl-androsta-1,4-dien-3,17-dion.

3. 15α-(1-Acetoxyethyl)-1-methyl-androsta-1,4-dien-3,17-dion,
15α-(1S-Acetoxyethyl)-1-methyl-androsta-1,4-dien-3,17-dion,
15α-(1R-Acetoxyethyl)-1-methyl-androsta-1,4-dien-3,17-dion,
15α-(1-Hydroxyethyl)-1-methyl-androsta-1,4-dien-3,17-dion,
15α-(1S-Hydroxyethyl-1-methyl-androsta-1,4-dien-3,17-dion,
15α-(1-Butyryloxyethyl)-1-methyl-androsta-1,4-dien-3,7-dion, und
15α-(1S-Butyryloxyethyl)-1-methyl-androsta-1,4-dien-3,17-dion.

4. 15α-Acetoxymethyl-1-methyl-androsta-1,4-dien-3,17-dion,
15α-Hydroxymethyl-1-methyl-androsta-1,4-dien-3,17-dion,
1-Methyl-15α-valeryloxymethyl-androsta-1,4-dien-3,17-dion und
1-Methyl-15α-(1S-valeryloxyethyl)-androsta-1,4-dien-3,17-dion.

5. 1,15α-Dimethyl-androsta-1,4-dien-3,17-dion.

6. 1-Methyl-15α-(1,2-isopropylidendioxypropyl)-androsta-1,4-dien-3,17-dion und
1-Methyl-15α-(1,2-dihydroxypropyl)-androsta-1,4-dien-3,17-dion.

7. 15α-(1-Acetoxybutyl)-1-methyl-androsta-1,4-dien-3,17-dion,
15α-(2-Acetoxybutyl)-1-methyl-androsta-1,4-dien-3,17-dion,
15α-(1S-Acetoxypropyl)-1-methyl-androsta-1,4-dien-3,17-dion und
15α-(1S-Acetoxybutyl)-1-methyl-androsta-1,4-dien-3,17-dion.

8. Pharmazeutische Präparate, gekennzeichnet durch den Gehalt an einer Verbindung gemäß Anspruch 1 bis 7.

9. Verwendung der Verbindungen gemäß Anspruch 1 bis 7 zur Herstellung von Präparaten zur Behandlung von östrogen bedingten Krankheiten.

10. Verfahren zur Herstellung von 1-Methyl-15α-alkyl-androsta-1,4-dien-3,17-dionen der allgemeinen Formel I

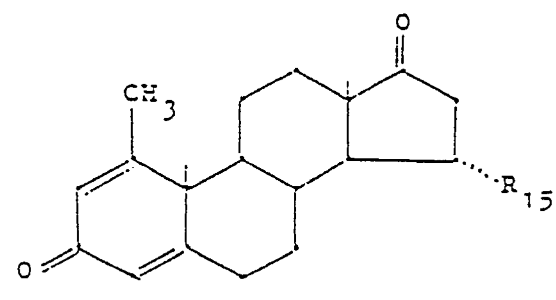

(I)

worin $R_{15}$ eine Alkylgruppe mit 1 bis 4 Kohlenstoffatomen darstellt, die gegebenenfalls in 1- und/oder 2-Stellung durch eine oder zwei Hydroxy- oder $C_2$-$C_7$-Alkanoyloxygruppen oder eine 1,2-Isopropylidendioxygruppe substituiert ist,
dadurch gekennzeichnet, daß man das 17-Hydroxysteroid der allgemeinen Formel II

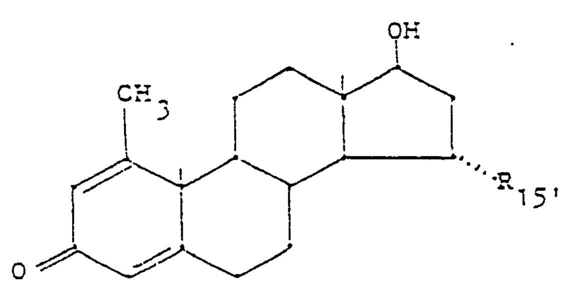

(II)

worin $R_{15'}$ eine Alkylgruppe mit 1 bis 4 Kohlenstoffatomen darstellt, die gegebenenfalls in 1- und/oder 2-Stellung durch eine oder zwei geschützte Hydroxygruppen substituiert ist, in an sich bekannter Weise oxydiert, gegebenenfalls Schutzgruppen abspaltet und gegebenenfalls Monohydroxygruppen alkanoyliert oder Dihydroxygruppen mit Aceton umsetzt.

## Claims

1. 1-Methyl-15α-alkylandrosta-1,4-diene-3,17-diones of the general formula I

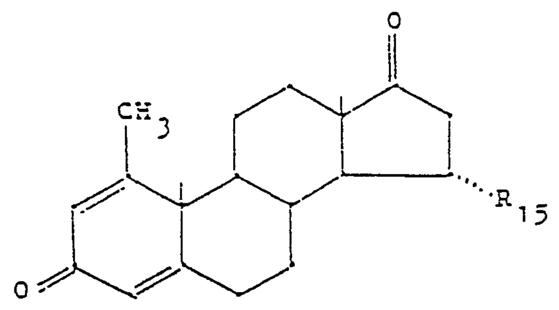

(I)

in which $R_{15}$ is an alkyl group of 1 to 4 carbon atoms, which may be substituted in the 1 and/or 2 position by a hydroxy or a $C_2$-$C_7$-alkanoyloxy group or a 1,2-isopropylidenedioxy group.

2. 15α-Ethyl-1-methylandrosta-1,4-diene-3,17-dione,
1-methyl-15α-propylandrosta-1,4-diene-3,17-dione and
15α-butyl-1-methylandrosta-1,4-diene-3,17-dione.

3. 15α-(1-Acetoxyethyl)-1-methylandrosta-1,4-diene-3,17-dione,
15α-(1S-acetoxyethyl)-1-methylandrosta-1,4-diene-3,17-dione,
15α-(1-hydroxyethyl)-1-methylandrosta-1,4-diene-3,17-dione,
15α-(1R-acetoxyethyl)-1-methylandrosta-1,4-diene-3,17-dione,
15α-(1S-hydroxyethyl)-1-methylandrosta-1,4-diene-3,17-dione,
15α-(1-butyryloxyethyl)-1-methylandrosta-1,4-diene-3,17-dione and
15α-(1S-butyryloxyethyl)-1-methylandrosta-1,4-diene-3,17-dione.

4. 15α-Acetoxymethyl-1-methylandrosta-1,4-diene-3,17-dione,
15α-hydroxymethyl-1-methylandrosta-1,4-diene-3,17-dione
1-methyl-15α-valeryloxymethylandrosta-1,4-diene-3,17-dione and
1-methyl-15α-(1S-valeryloxyethyl)-androsta-1,4-diene-3,17-dione.

5. 1,15α-Dimethylandrosta-1,4-diene-3,17-dione.

6. 1-Methyl-15α-(1,2-isopropylidenedioxypropyl)-androsta-1,4-diene-3,17-dione and
1-methyl-15α-(1,2-dihydroxypropyl)-androsta-1,4-diene-3,17-dione.

7. 15α-(1-Acetoxybutyl)-1-methylandrosta-1,4-diene-3,17-dione,
15α-(2-acetoxybutyl)-1-methylandrosta-1,4-diene-3,17-dione,
15α-(1S-acetoxypropyl)-1-methylandrosta-1,4-diene-3,17-dione and
15α-(1S-acetoxybutyl)-1-methylandrosta-1,4-diene-3,17-dione.

8. Pharmaceutical compositions, characterised in that they contain a compound according to any one of Claims 1 to 7 in admixture with a pharmaceutically acceptable diluent or carrier.

9. Use of the compounds according to any one of Claims 1 to 7 for the production of compositions for the treatment of illnesses caused by estrogen.

10. Process for the preparation of 1-methyl-15α-alkyl-androsta-1,4-diene-3,17-diones of the general formula I

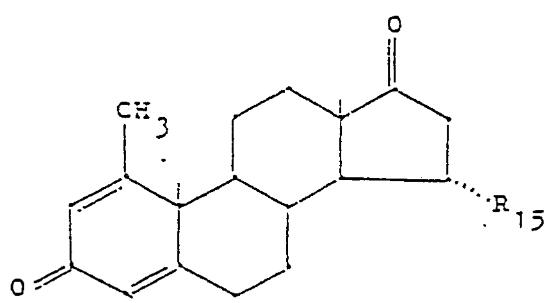

(I)

in which $R_{15}$ is an alkyl group of 1 to 4 carbon atoms, which may be substituted in the 1 and/or 2 position by a hydroxy or a $C_2$-$C_7$-alkanoyloxy group or a 1,2-isopropylidenedioxy group,

characterized in that the 17-hydroxy-steroid of the general formula II

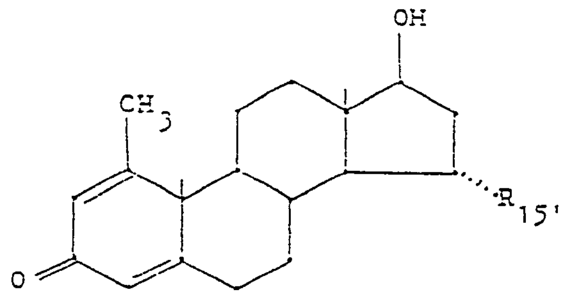

(II)

in which $R_{15'}$ represents an alkyl group of 1 to 4 carbon atoms, which may be substituted in the 1 and/or 2-position by one or two protected hydroxy groups, is oxidized in a known manner, if necessary the protecting groups are removed and if necessary the monohydroxy groups are alkanoylated or the dihydroxy groups are reacted with acetone.

**Revendications**

1. Méthyl-1 alkyl-15α androstadiène-1,4 diones-3,17 qui répondent à la formule générale I :

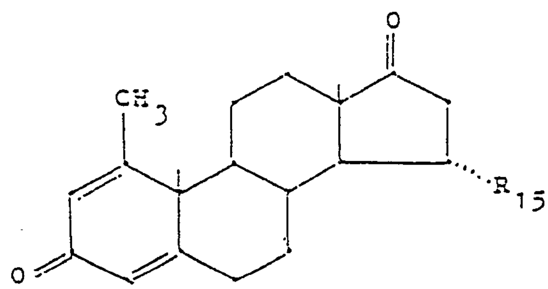

(I)

dans laquelle $R_{15}$ représente un radical alkyle qui contient de 1 à 4 atomes de carbone et qui porte éventuellement, en position 1 et/ou en position 2, un ou deux radicaux hydroxy ou alcanoyloxy en $C_2$-$C_7$ ou un radical isopropylidène-dioxy en 1,2.

2. Ethyl-15α méthyl-1 androstadiène-1,4 dione-3,17,
propyl-15α méthyl-1 androstadiène-1,4 dione-3,17 et
butyl-15α méthyl-1 androstadiène-1,4 dione-3,17.

3. (Acétoxy-1 éthyl)-15α méthyl-1 androstadiène-1,4 dione-3,17,
(acétoxy-1S éthyl)-15α méthyl-1 androstadiène-1,4 dione-3,17,
(acétoxy-1R éthyl)-15α méthyl-1 androstadiène-1,4 dione-3,17,
(hydroxy-1 éthyl)-15α méthyl-1 androstadiène-1,4 dione-3,17,
(hydroxy-1S éthyl)-15α méthyl-1 androstadiène-1,4 dione-3,17,
(butyryloxy-1 éthyl)-15α méthyl-1 androstadiène-1,4 dione-3,17 et
(butyryloxy-1S éthyl)-15α méthyl-1 androstadiène-1,4 dione-3,17.

4. Acétoxyméthyl-15α méthyl-1 androstadiène-1,4 dione-3,17,
hydroxyméthyl-15α méthyl-1 androstadiène-1,4 dione-3,17,
méthyl-1 valéryloxyméthyl-15α androstadiène-1,4 dione-3,17 et
méthyl-1 (valéryloxy-1S éthyl)-15α androstadiène-1,4 dione-3,17.

5. Diméthyl-1,15α androstadiène-1,4 dione-3,17.

6. Méthyl-1 (isopropylidène-dioxy-1,2 propyl)-15α androstadiène-1,4 dione-3,17 et
méthyl-1 (dihydroxy-1,2 propyl)-15α androstadiène-1,4 dione-3,17.

7. (Acétoxy-1 butyl)-15α méthyl-1 androstadiène-1,4 dione-3,17,
(acétoxy-2 butyl)-15α méthyl-1 androstadiène-1,4 dione-3,17,
(acétoxy-1S propyl)-15α méthyl-1 androstadiène-1,4 dione-3,17 et
(acétoxy-1S butyl)-15α méthyl-1 androstadiène-1,4 dione-3,17.

8. Compositions pharmaceutiques caractérisées en ce qu'elles contiennent un composé selon l'une quelconque des revendications 1 à 7.

9. Application des composés selon l'une quelconque des revendications 1 à 7 à la fabrication de compositions pour le traitement de maladies conditionnées par des œstrogènes.

10. Procédé de préparation de méthyl-1 alkyl-15α androstadiène-1,4 diones-3,17 qui répondent à la formule générale I :

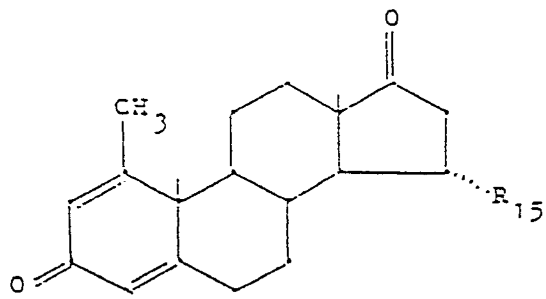

(I)

dans laquelle $R_{15}$ représente un radical alkyle qui contient de 1 à 4 atomes de carbone et qui porte éventuellement, en position 1 et/ou en position 2, un ou deux radicaux hydroxy ou alcanoyloxy en $C_2$-$C_7$ ou un radical isopropylidène-dioxy en 1,2,
procédé caractérisé en ce qu'on oxyde, de manière connue, l'hydroxy-17 stéroïde qui répond à la formule générale II :

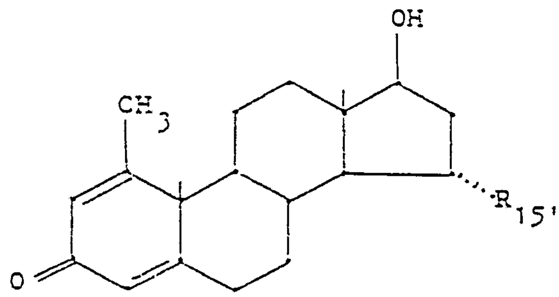

(II)

dans laquelle $R_{15'}$ représente un radical alkyle qui contient de 1 à 4 atomes de carbone et qui porte éventuellement, en position 1 et/ou en position 2, un ou deux radicaux hydroxy protégés, on élimine éventuellement des radicaux protecteurs et éventuellement on alcanoyle des radicaux monohydroxy ou on fait réagir des radicaux dihydroxy avec l'acétone.